(19) Europäisches Patentamt
European Patent Office
Office européen des brevets

(11) Veröffentlichungsnummer : **0 292 434 B1**

(12) # EUROPÄISCHE PATENTSCHRIFT

(45) Veröffentlichungstag der Patentschrift : **08.07.92 Patentblatt 92/28**

(51) Int. Cl.$^5$ : **C07D 403/06,** C07D 403/10, C07D 403/12, C08G 73/12

(21) Anmeldenummer : **88810308.2**

(22) Anmeldetag : **11.05.88**

(54) **Ungesättigte Bisimide und deren Polymeren.**

Die Akte enthält technische Angaben, die nach dem Eingang der Anmeldung eingereicht wurden und die nicht in dieserPatentschrift enthalten sind.

(30) Priorität : **22.05.87 CH 1992/87**

(43) Veröffentlichungstag der Anmeldung : **23.11.88 Patentblatt 88/47**

(45) Bekanntmachung des Hinweises auf die Patenterteilung : **08.07.92 Patentblatt 92/28**

(84) Benannte Vertragsstaaten : **CH DE FR GB IT LI NL**

(56) Entgegenhaltungen :
**EP-A- 0 105 024**
**EP-A- 0 152 372**
**EP-A- 0 175 648**
**CHEMICAL ABSTRACTS, Band 108, Nr. 7, 15. Februar 1988, Columbus, Ohio, US; MUSTAFAEV, A.M.; GUSEINOV, M.M.; GASANOVA, K.M.;GADZHIEVA, S.T.: "Polybromocyclopentadienes in the Diels-Alder reaction. VI. Reactivity of hexabromo-and5,5-dimethoxytetrabromo-1,3-cyclopentadienes in the Diels-Alder reaction with bismaleimides." Seite 648, Spalte 2,Zusammenfassung-Nr. 55 230d**
**CHEMICAL ABSTRACTS, Band 74, Nr. 11, 15. März 1971, Columbus, Ohio, US; KAMENSKAYA, S.A.; SEMENOVA, L.P.; EITINGON, I.I.:"New 1,4-bis(dicarboximidomehtyl) derivatives of piperazine and their infrared spectra." Seite 361, Spalte 2, Zusammenfassung-Nr.53 729d**
**CHEMICAL ABSTRACTS, Band 100, Nr. 19, 7. Mai 1984, Columbus, Ohio, US; AMATI, WERNER: "Amphoteric and anionic sufactant andits use." Seite 513, Spalte 1, Zusammenfassung-Nr. 156 371r**

(56) Entgegenhaltungen :
**CHEMICAL ABSTRACTS, Band 94, Nr. 12, 23. März 1981, Columbus, Ohio, US; SCOLA, DANIEL A.; STEVENS, MALVOLM P.: "Synthesis andpolymerization of aliphatic bisnadimides." Seite 31, Spalte 1, Zusammenfassung-Nr. 84 922d**

(73) Patentinhaber : **CIBA-GEIGY AG**
**Klybeckstrasse 141**
**CH-4002 Basel (CH)**

(72) Erfinder : **Kramer, Andreas, Dr.**
**Bundtels**
**CH-3186 Düdingen (CH)**
Erfinder : **Eldin, Sameer H., Dr.**
**Route de Schiffenen 10**
**CH-1700 Fribourg (CH)**

EP 0 292 434 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft gegebenenfalls substituierte Bicyclo[2.2.1]-hept-5-en-2,3-dicarbonsäure/Maleinsäure gemischte Bisimide, deren Herstellung, die Bisimide enthaltenden Stoffgemische und die daraus durch Erwärmen erhältlichen Polymeren.

Die US 4,515,962 und US 4,604,437 beschreiben allyl- oder methallyl-substituierte Bicyclo[2.2.1]hept-5-en-2,3-dicarbonsäureimide und deren Verwendung zur Herstellung von Polymeren durch Erhitzen der Imide auf Temperaturen zwischen 180 und 300°C.

Es ist auch bekannt, dass sich Polymaleinimide mit oder ohne Zusatz von Vernetzungsmitteln, wie Diaminen oder Diolen, durch Erhitzen in vernetzte Produkte überführen lassen [vgl. z.B. US 3,562,223, US 3,658,764, US 3,380,964 und US 4,038,251].

Die US 4,666,997 beschreibt heisshärtbare Stoffgemische aus substituierten Bicyclo[2.2.1]hept-5-en-2,3-dicarbonsäureimiden, Polymaleinimiden und gegebenenfalls zusätzlich einer zur Reaktion mit den Polymaleinimiden befähigten Komponente.

In Zh. Org. Chim. 23, 5 (1987) werden ausserdem auf Seite 964 fünf hexabromierte Verbindungen enthaltend eine Bicyclo[2.2.1]hept-5-en-2,3-dicarbonsäureimidegruppe und eine Maleinimidgruppe auf der Basis von para-Phenylen oder Diphenylmethan, -oxid, -sulfid oder -sulfon beschrieben.

Gegenstand vorliegender Erfindung sind Bisimide der Formel I

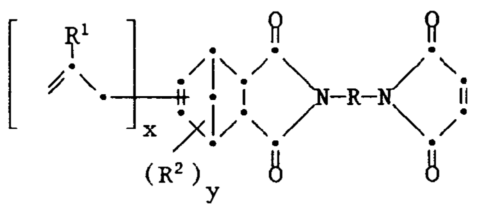

(I) ,

worin x Null, 1, 2 oder 3, y Null oder eine ganze Zahl von 1 bis 6 und $x + y \leqq 6$ sind, $R^1$ Wasserstoff oder Methyl und $R^2$ Chlor, Brom, $C_1$-$C_4$-Alkyl oder Benzyl bedeuten und R für $-C_m H_{2m}-$ mit m = 2-20, Cycloalkylen mit 5 bis 10 C-Atomen, Bis(methylen)cycloalkylen mit 7 bis 12 C-Atomen, Arylen mit 6 bis 10 C-Atomen, Bis(methylen)arylen mit 8 bis 12 C-Atomen oder für eine Gruppe der Formel II

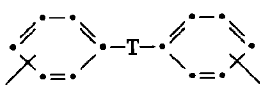

(II) ,

worin T Methylen, Isopropyliden, CO, O, S oder $SO_2$ bedeutet, steht, wobei Bisimide der Formel I, in welcher x Null, y 6, $R^2$ Brom und R para-Phenylen oder eine Gruppe der Formel II sind, worin T O, S, $SO_2$ oder $-CH_2-$ bedeutet, ausgeschlossen sind.

Die erfindungsgemässen Bisimide sind wertvolle Ausgangsprodukte für Polymere mit ausgezeichneten Eigenschaften und eignen sich zur Herstellung von Formkörpern, Beschichtungen und Verklebungen und insbesondere für die Herstellung von Prepregs und hitzebeständigen Verbundwerkstoffen.

Bevorzugt werden Bisimide der Formel I, worin $x + y \leqq 4$ sind und insbesondere solche, worin $x + y \leqq 2$ sind.

Bevorzugt werden Bisimide der Formel I, worin $R^1$ Wasserstoff, x 1 und y Null bedeuten. Bevorzugte Bisimide der Formel I sind auch solche, worin $R^2$ $C_1$-$C_3$-Alkyl oder Benzyl, y 1 und x Null bedeuten.

Wenn der Substituent $R^2$ der erfindungsgemässen Bisimide $C_1$-$C_4$-Alkyl bedeutet, kommen Methyl, Ethyl, n- und i-Propyl und n-, i-, sec. und tert.-Butyl in Frage.

Stellt R eine Gruppe $-C_m H_{2m}-$ dar, so kann es sich um geradkettige oder verzweigte Reste handeln, wie Ethylen, Propylen, Trimethylen, Tetramethylen, Hexamethylen, Octamethylen und Dodecemethylen.

Bedeutet R eine Gruppe der Formel II, so ist diese vorzugsweise in 4,4'-Stellung an die N-Atome gebunden. Wenn R eine Cycloalkylengruppe bedeutet, kann es sich um Cyclopentylen-, Cyclohexylen-, Cycloheptylen- oder Cyclooctylengruppen, um Bis(cycloalkylen)methangruppen oder auch um bicyclische Systeme, wie z.B. Decalinylen- oder Bicycloheptylen-oder Bicyclooctylengruppen handeln. Wenn R ein Bis(methylen)cycloalkylenrest ist, kommen die oben erwähnten Cycloalkylengruppen in Frage, welche durch zwei Methylengruppen substituiert sind, wie z.B. Bis(methylen)cyclohexan.

R kann in der Bedeutung einer Arylengruppe mit 6 bis 10 C-Atomen z.B. eine m-Phenylen, p-Phenylen, 1,3-Naphthylen-, 1,4-Naphthylen-, 1,5-Naphthylen-oder 2,6-Naphthylengruppe sein. Wenn R für einen Bis(me-

thylen)arylenrest steht, kommen die oben erwähnten Arylengruppen in Frage, welche durch zwei Methylengruppen substituiert sind.

Bevorzugt werden Bisimide der Formel I, worin R $-(CH_2)_p-$ mit p = 2-12, Cyclohexylen, Bis(methylen)cyclohexan, Bis(cyclohexylen)methan, Phenylen, wie 1,2-, 1,3- und 1,4-Phenylen, Xylylen, wie 1,2-, 1,3- und 1,4-Xylylen, oder insbesondere eine Gruppe der Formel II bedeutet, worin T für Methylen, O oder $SO_2$ steht.

Besonders bevorzugt sind Bisimide der Formel I, worin R 1,4-Phenylen, 1,4-Xylylen oder Bis(1,4-phenylen)methan bedeutet.

Die erfindungsgemässen Bisimide können durch eine Diels-Alder Reaktion eines Bismaleinimids der Formel III

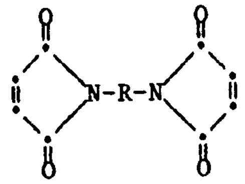 (III)

mit einem Cyclopentadien der Formel IV

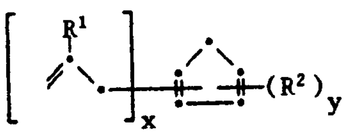 (IV)

hergestellt werden, wobei die Symbole R, $R^1$, $R^2$, x und y die oben angegebene Bedeutung haben. Die Umsetzung erfolgt vorzugsweise in einem inerten, für das Bismaleinimid der Formel (III) geeigneten Lösungsmittel, wie z.B. Dioxan, Tetrahydrofuran, Aceton, Methylenchlorid, Chloroform oder Toluol, indem z.B. das Cyclopentadien der Formel IV in eine Lösung des Bismaleinimids bei ca. 20 bis 150°C zugetropft wird, das Reaktions gemisch 1 bis 5 Stunden nachgerührt wird und das gewünschte Diels-Alder Addukt durch Entfernung des Lösungsmittels isoliert wird. Bei der beschriebenen Umsetzung entstehen im allgemeinem neben dem erfindungsgemässen Bisimid der Formel I auch symmetrische Bisimide, bei denen beide Maleinimidylreste des Bismaleinimids der Formel III mit dem Cyclopentadien der Formel IV reagiert haben, so dass beim äquimolaren Einsatz der Edukte der Formeln III und IV im Produktegemisch auch noch nicht umgesetztes Bismaleinimid der Formel III vorliegt. Aus den so erhaltenen Produktegemischen können die Bisimide der Formel I, z.B. mit HPLC, in reiner Form isoliert werden. Die erwähnten Produktegemische können aber ohne weiteres direkt für die Herstellung von wertvollen vernetzten Polymeren eingesetzt werden.

Die Edukte der Formeln III und IV sind bekannt und können auf bekannte Weise hergestellt werden. Bismaleinimide der Formel III sind z.B. in den US 3,562,223, US 3,658,764, US 3,380,964 und US 4,038,251 beschrieben und Cyclopentadiene der Formel IV sind z.B. in US 3,560,583 offenbart.

Die erfindungsgemässen Bisimide der Formel I können falls erwünscht auch in reiner Form, z.B. wie folgt, hergestellt werden:

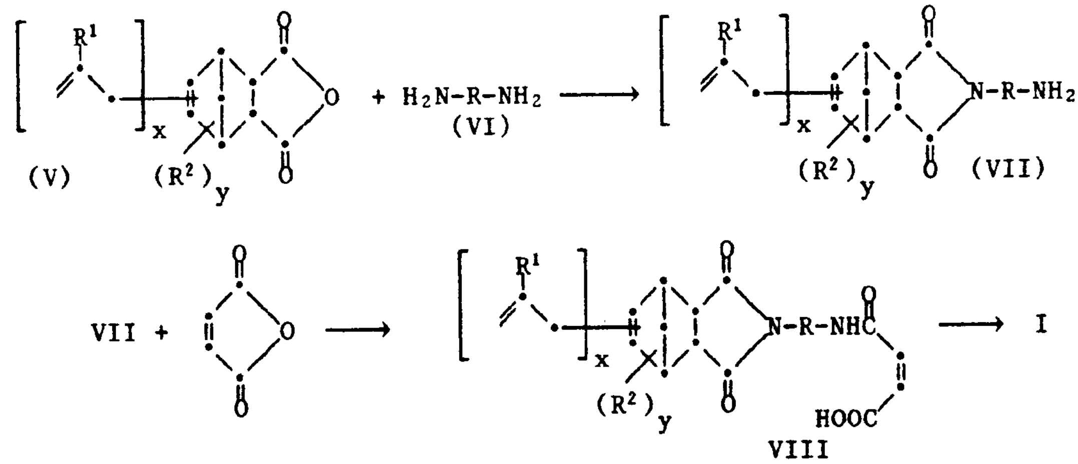


Im ersten Reaktionsschritt wird ein substituiertes Bicyclo[2.2.1]hept-5-en-2,3-dicarbonsäureanhydrid der Formel V mit einem Diamin der Formel VI umgesetzt und das Monoimid-Monoamin VII wird aus dem Reaktionsgemisch isoliert. Im zweiten Reaktionssschritt wird die Verbindung VII mit Maleinsäureanhydrid über die entsprechende Amidsäure der Formel VIII zum gewünschten erfindungsgemässen Bisimid I umgesetzt.

Die Zeichen $R^1$, $R^2$, R, x und y haben in den Verbindungen der Formeln V bis VIII die oben angegebene Bedeutung.

Anhydride der Formel V sind bekannt und können z.B. durch Diels-Alder Addition von Cyclopentadienen der Formel IV mit Maleinsäureanhydrid hergestellt werden. Solche Verbindungen und deren Herstellung sind in US 3,105,839 beschrieben.

Gegenstand der Erfindung sind auch Stoffgemische enthaltend ein Bisimid der Formel I, erhältlich durch Umsetzung eines Bismaleinimids der Formel III

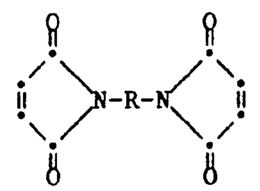

(III)

mit 0,4 bis 1,6 Aequivalenten eines Cyclopentadiens der Formel IV

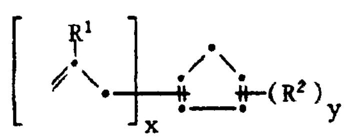

(IV),

wobei die Symbole R, $R^1$, $R^2$, x und y die oben angegebene Bedeutung haben.

Somit enthalten die erfindungsgemässen Stoffgemische mindestens 20 Mol% eines erfindungsgemässen Bisimids der Formel I, wobei die übrigen Komponenten der Stoffgemische das nicht umgesetzte Bismaleinimid der Formel III sowie das durch Umsetzung beider Maleinimidgruppen des Imids der Formel III entstandene Bis(bicyclo[2.2.1]hept-5-en-2,3-dicarbonsäureimid) sind.

Bevorzugt werden Stoffgemische, worin pro Bismaleinimid der Formel III 0,7 bis 1,3, vorzugsweise 1, Aequivalent des Cyclopentadiens der Formel IV eingesetzt werden.

Die erfindungsgemässen Stoffgemische können, falls zweckmässig, zusätzlich 1 bis 30, vorzugsweise 5 bis 25, Gew.-%, bezogen auf das Gesamtgemisch, einer zur Reaktion mit dem Bisimid der Formel I oder mit dem Bismaleinimid der Formel III befähigten Verbindung enthalten. Es können dabei beliebige bekannte Verbindungen eingesetzt werden. Verbindungen, die mit Bismaleinimiden oder mit der Maleinimidgruppe der Bisimide der Formel I reagieren können, sind zum Beispiel in der US 4,666,997 als Komponente (c) der Stoffgemische beschrieben. Besonders geeignet sind dabei Diamine und Diole, sowie Phenol- und Kresol-Novolake oder Gemische solcher Verbindungen.

Besonders geeignet sind Diamine und Diole der Formel IX

$$HQ\text{-}R'\text{-}QH \quad (IX)$$

worin Q O oder NH und R′ einen zweiwertigen organischen Rest mit 2-30 C-Atomen bedeuten, sowie Phenol- und Kresol-Novolake oder Gemische solcher Verbindungen.

R′ ist als zweiwertiger organischer Rest vorzugsweise $-C_pH_{2p}-$ mit p = 2-20, besonders $-(CH_2)_p-$ mit p = 2-12, Arylen mit 6-10 C-Atomen, besonders m- oder p-Phenylen, Xylylen, Cyclopentylen, Cyclohexylen, 1,4-Bis(methylen)cyclohexylen, der Rest des Bicyclohexylmethans oder ein Rest der Formel II. Besonders bevorzugt ist R′ ein in 4,4′-Stellung gebundener Rest der Formel II.

Eine weitere Klasse bevorzugter Verbindungen sind alkenylsubstituierte Phenole und Polyole, wie zum Beispiel Verbindungen der Formel X

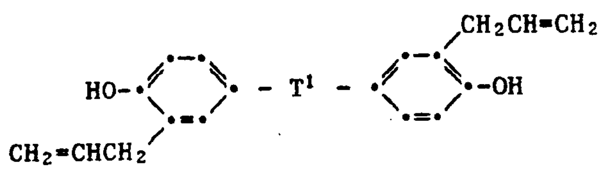

(X),

worin $T^1$ die direkte Bindung, Methylen, Isopropyliden, O, S, SO oder $SO_2$ bedeutet. Beispiele solcher Verbindungen sind: Bis(4-hydroxy-3-allyl)biphenyl, Bis(4-hydroxy-3-allylphenyl)methan und 2,2-Bis(4-hydroxy-3-allylphenyl)propan (o,o′-Diallyl-bisphenol A).

Bevorzugt enthalten die erfindungsgemässen Stoffgemische als zur Reaktion mit den Bisimiden der Formel I oder den Bismaleinimiden der Formel III befähigte Verbindungen Phenol- oder Kresol-Novolake, Verbindungen der Formel IXa

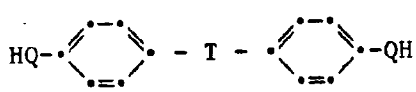

(IXa),

worin Q und T die oben unter der Formel IX bzw. II angegebene Bedeutung haben und T insbesondere Methylen oder Isopropyliden darstellt, oder Verbindungen der Formel X, worin $T^1$ Isopropyliden ist, sowie Gemische der genannten bevorzugten Verbindungen. Die Verbindungen der Formeln IX, IXa und X sind bekannt oder können nach an sich bekannten Methoden hergestellt werden.

Als Verbindungen, welche mit der Bicyclo[2.2.1]hept-5-en-2,3-dicarbonsäureimidgruppe der Bisimide der Formel I reagieren können, eignen sich insbesondere die in der EP-A 190 102 beschriebenen sulfonyloxysgruppenhaltigen bicyclischen Imide der Formel XI

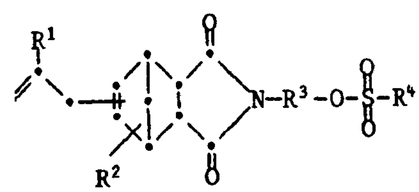

(XI),

wobei $R^1$, $R^2$, $R^3$ und $R^4$ die in der EP-A 190 102 angegebene Bedeutung haben.

Die erfindungsgemässen Bisimide sind Ausgangstoffe für die Herstellung von Polymeren mit ausgezeichneten physikalischen Eigenschaften.

Ein weiterer Gegenstand der Erfindung sind Polymere, die dadurch erhältlich sind, dass man ein Bisimid der Formel I während 1 bis 20 Stunden auf eine Temperatur zwischen 100 und 300°C erhitzt.

Die Härtung bzw. Verarbeitung der Bisimide der Formel I und der sie enthaltenden Stoffgemische kann in einem inerten organischen Lösungsmittel, vorzugsweise jedoch aus der Schmelze und gegebenenfalls in Gegenwart eines Härtungskatalysators erfolgen. Als inerte organische Lösungsmittel eignen sich z.B. Dimethylformamid, Dimethylacetamid, N-Methylpyrrolidon, Toluol, Xylole, Methylenchlorid, Tetrahydrofuran, Methylethylketon, und Ethylenglykolmonoalkyl- oder -dialkylether mit 1-4 C-Atomen in den Alkylgruppen. Je

nach der beabsichtigten Verwendung kommen als Härtungskatalysatoren z.B. organische Peroxide, wie Ditert-butylperoxid, Dicumylperoxid, tert-Butylperbenzoat, oder basische Katalysatoren, vor allem primäre, sekundäre und tertiäre Amine in Betracht, wie z.B. Diethylamin, Tributylamin, Triethylamin, Benzylamin, N,N,N',N'-Tetramethyl-4,4'-diaminodiphenylmethan, N,N-Diisobutylaminoacetonitril, N,N-Dibutylaminoacetonitril und heterocyclische Basen, wie Chinolin, N-Methylpyrrolidin und Imidazol. Besonders bei der Verwendung von Phenolen oder Polyolen als zusätzliche, zur Reaktion mit den Maleinimidgruppen befähigte Komponente der erfindungsgemässen Stoffgemische empfiehlt sich der Zusatz von basischen Katalysatoren der erwähnten Art. Die Härtung wird im allgemeinen bei Temperaturen zwischen 100 und 300°C, besonders 120 und 250°C vorgenommen.

Die erfindungsgemässen Bisimide und sie enthaltende Stoffgemische stellen niederschmelzende Festharze bis viskose Flüssigharze dar und zeichnen sich durch hohe Reaktivität und gute mechanische Eigenschaften der damit gehärteten Produkte aus, wie gute Biege- und Schlagbiegefestigkeit. So erhaltene Produkte weisen hohe Glasumwandlungstemerpaturen und eine sehr hohe thermische Beständigkeit auf und sind zudem auch wenig spröde. Die erfindungsgemässen Bisimide und Stoffgemische können ferner leicht aus der Schmelze, besonders auch ohne Zusatz von schwerflüchtigen Lösungsmittlen, appliziert werden, beispielsweise zum Imprägnieren von Glasfaser-, Kohlenstoffaser- oder Aramidfaser-Gewebe, wie Fasergewebe aus den unter dem Handelsnamen Kevlar® bekannten Poly(1,4-phenylenterephthalamiden).

Die erfindungsgemässen Bisimide und sie enthaltende Stoffgemische können vielseitig angewendet werden, z.B. als Laminier- oder Elektroharze, als Hochtemperatur-Klebstoffe oder zur Herstellung von Beschichtungen oder Formkörpern. Ganz besonders geeignet sind sie für die Herstellung von Prepregs und hitzebeständigen Verbundwerkstoffen.

Gegenstand der Erfindung ist auch die Verwendung der Bisimide der Formel I zur Herstellung von Formkörpern, Prepregs, Laminaten, Beschichtungen oder Verklebungen.

Die erfindungsgemässen gemischten (unsymetrischen) Bisimide der Formel I weisen überraschenderweise eine höhere Reaktivität bei der Polymerisation auf als sowohl die symmetrischen Bismaleinimide bzw. die symmetrischen Bis(bicyclo[2.2.1]hept-5-en-2,3-dicarbonsäureimide) als auch Gemische dieser symmetrischen Bisimide. Zudem zeichnen sich die mit den erfindungsgemässen Bisimiden der Formel I bzw. den sie enthaltenden erfindungsgemässen Stoffgemischen durch Vernetzung hergestellten Polymere durch wesentlich höhere Glasumwandlungstemperaturen aus.

Die Erfindung wird durch die folgenden Beispiele näher erläutert.

Beispiel 1: <u>Herstellung des mono-Diels-Alder Addukts von Allylcyclopentadien und N,N',4,4'-Diphenylmethan-bismalein-imid</u>

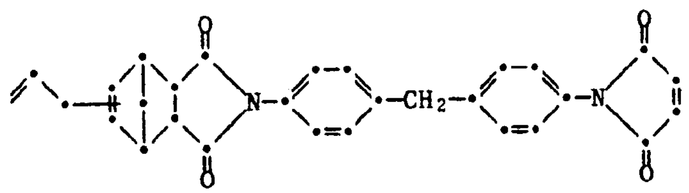

Zu einer Lösung von 358 g N,N',4,4,'-Diphenylmethanbismaleinimid in 800 ml Dioxan tropft man bei 60°-65°C innert 30 Minuten 132 g Allylcyclopentadien (hergestellt gemäss US-PS 3,560,583, Beispiel 1) zu und rührt anschliessend zwei Stunden bei 60°C nach. Die klare, rotgelbe Reaktionslösung wird abgekühlt, das Lösungsmittel im Wasserstrahlvakuum abdestilliert und der Rückstand 2 Stunden im Hochvakuum bie 80°C getrocknet. Man erhält 485 g (99 % der Theorie) eines gelbroten Festharzes mit einem Erweichungspunkt von 57°C und einem durch Gelpermeationschromatographie (THF) ermittelten Molekulargewicht von 452 ($\overline{M}_n$) bzw. 472 ($\overline{M}_w$). Im IR-Spektrum findet sich eine Absorptionsbande bei 1710 $cm^{-1}$ (>C = O) und bei 1640 $cm^{-1}$ (>C=C<).

<u>Analyse</u>

| | % C | % H | % N |
|---|---|---|---|
| berechnet für $C_{29}H_{24}N_2O_4$ | 74,98 | 5,21 | 6,03 |
| gefunden | 74,84 | 5,34 | 5,79 |

Beispiel 2: Herstellung des mono Diels-Alder Addukts von Allylcyclopentadien und N,N',4,4'-Diphenylether-bismaleinimid

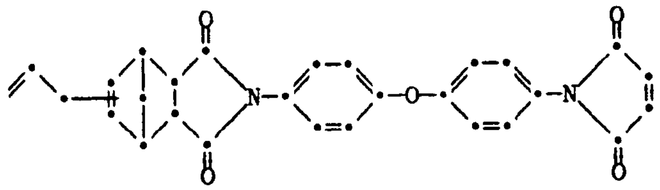

Verwendet man anstelle der in Beispiel 1 eingesetzten Bismaleinimidverbindung das N,N',4,4'-Diphenylether-bismaleinimid und geht im übrigen in gleicher Weise wie in Beispiel 1 beschrieben vor, so erhält man in quantitativer Ausbeute ein rotbraunes Festharz mit einem Erweichungspunkt von 56°C und einem durch Gelpermeationschromatographie (THF) ermittelten Molekulargewicht von 589 ($\overline{M}_n$) bzw. 6103 ($\overline{M}_w$).

Analyse

| | %C | %H | %N |
|---|---|---|---|
| berechnet für $C_{28}H_{22}N_2O_5$ | 72,09 | 4,75 | 6,00 |
| gefunden | 71,76 | 5,20 | 5,30 |

Beispiel 3: Herstellung des mono Diels-Alder Addukts von Allylcyclopentadien und N,N',4,4'-Diphenylsulfon-bismaleinimid

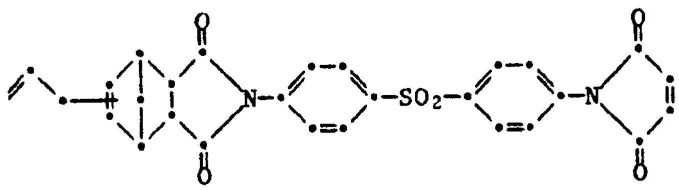

Man verfährt wie in Beispiel 1, verwendet aber das Bismaleinimid des N,N'4,4'-Diaminodiphenylsulfons. Man erhält ein rotbraunes Festharz mit einem Erweichungspunkt von 49°C und einem durch Gelpermeationschromatographie (THF) ermittelten Molekulargewicht von 763 ($\overline{M}_n$) bzw. 2826 ($\overline{M}_w$).

Beispiel 4: Herstellung des mono-Diels-Alder Addukts von Allylcyclopentadien und N,N',1,6'-Hexamethylen-bismaleinimid

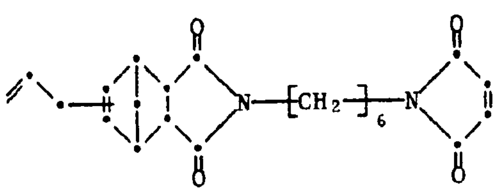

Zu einer Lösung von 1 Mol N,N',1,6-Hexamethylenbismaleinimid in 700 ml Dioxan tropft man bei 70°C innert 30 Min 1 Mol Allylcyclopentadien zu und rührt anschliessend zwei Stunden bei 70°C nach. Die klare Reaktionslösung wird abgekühlt und das Lösungsmittel am Rotationsverdampfer abdestilliert. Man isoliert in quantitativer Ausbeute ein flüssiges Harz das sich beim Stehenlassen verfestigt. Durch Gelpermeationschromatographie (THF) wurde ein Molekulargewicht von 440 ($\overline{M}_n$) bzw. 12160 ($\overline{M}_w$) bestimmt.

Analyse

| | % C | % H | % N |
|---|---|---|---|
| berechnet für $C_{22}H_{26}N_2O_4$ | 69,09 | 6,85 | 7,32 |
| gefunden | 69,23 | 6,85 | 6,99 |

Beispiel 5: Herstellung des mono Diels-Alder Addukts von Methylcyclopentadien und N,N',4,4'-Diphenylmethan-bismaleinimid

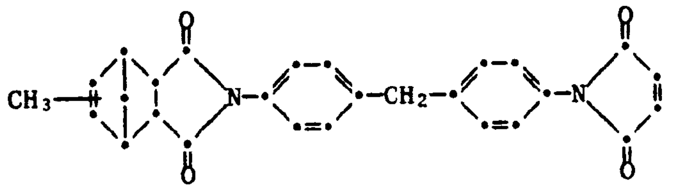

Zu einer Lösung von 179 g N,N',4,4'-Diphenylmethanbismaleinimid in 500 ml Dioxans tropft man bei 70°C innert 15 Minuten 40 g Methylcyclopentadien (hergestellt durch Cracken von Methylcylopentadiendimer) zu und rührt anschliessend zwei Stunden bei 60°C nach. Das Lösungsmittel wird abdestilliert und der klare, gelbe Rückstand 2 Stunden im Hochvakuum bei 120°C getrocknet. Man isoliert 212 g eines gelben Festharzes mit einem Erweichungspunkt von 65°C und einem durch Gelpermeationschromatographie (THF) ermittelten Molekulargewicht von 422 ($\overline{M}_n$) bzw. 660 ($\overline{M}_w$).

Analyse

| | % C | % H | % N |
|---|---|---|---|
| berechnet für $C_{27}H_{22}N_2O_4$ | 73,96 | 5,06 | 6,39 |
| gefunden | 73,55 | 5,18 | 6,35 |

Beispiel 6: Herstellung des mono-Diels-Alder Addukts von Propylcyclopentadien und N,N',4,4'-Diphenylmethan-bismaleinimid

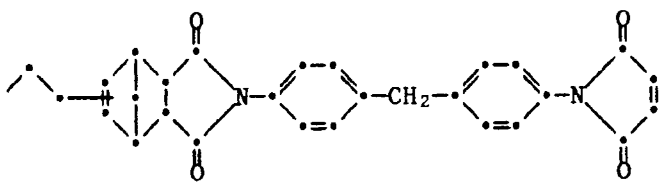

Zu einer Lösung von 179 g N,N',4,4'-Diphenylmethanbismaleinimid in 500 ml Dioxans tropft man bei 70°C innert 20 Minuten 56 g Propylcyclopentadien (hergestellt aus Cyclopentadien und Propylbromid gemäss US-PS 3,560,583) zu und rührt anschliessend zwei Stunden bei 60°C nach. Das Lösungsmittel wird im Wasserstrahlvakuum abdestilliert und der Rückstand eine Stunde im Hochvakuum bei 120°C getrocknet. Man erhält 227 g (97,5 % der Theorie) eines rötlichen Festharzes mit einem Erweichungspunkt von 60°C und einem durch Gelpermeationschromatographie (THF) ermittelten Molekulargewicht von 428 ($\overline{M}_n$) und 433 ($\overline{M}_w$).

| Analyse: | % C | % H | % N |
|---|---|---|---|
| berechnet für $C_{29}H_{26}N_2O_4$ | 74,66 | 5,62 | 6,00 |
| gefunden | 73,92 | 5,60 | 5,98 |

Beispiel 7: Herstellung des mono-Diels-Alder von Benzylcyclopentadien und N,N',4,4'-Diphenylmethan-bismaleinimid

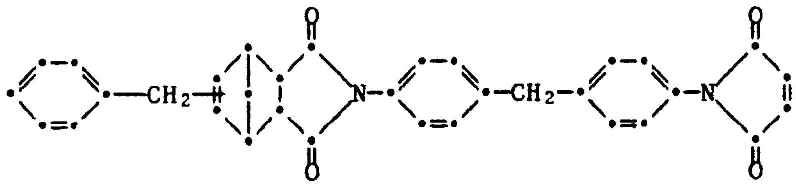

Zu einer Lösung von 125,3 g N,N',4,4'-Diphenylmethanbismaleinimid in 400 ml Dioxan tropft man bei 60°-65°C innert 10 Minuten 60 g Benzylcyclopentadien (hergestellt gemäss US-PS 3,560,583, Beispiel 3) zu und rührt anschliessend zwei Stunden bei 60°C nach. Das Lösungsmittel wird dann im Wasserstrahlvakuum abdestilliert und der Rückstand eine Stunde bei 120°C getrocknet. Man isoliert 175 g (97 % der Theorie) eines gelbroten Festharzes mit einem Erweichungspunkt von 67°C und einem durch Gelpermeationschromatographie (THF) ermittelten Molekulargewicht von 466 ($\overline{M}_n$) und 567 ($\overline{M}_w$).

| Analyse | % C | % H | % N |
|---|---|---|---|
| berechnet für $C_{33}H_{26}N_2O_4$ | 77,03 | 5,09 | 5,44 |
| gefunden | 76,83 | 5,24 | 5,23 |

Anwendungsbeispiele I-IV

Die gemäss der Beispielen 1, 5, 6 und 7 erhaltenen Bisimide werden als heisse, dünnflüssige Harze in Stahlformen von 12 x 12 x 0,4 $cm^3$ gegossen, während je 3 Stunden bei 160°C, 200°C und 220°C vorgeliert und anschliessend während 12 Stunden bei 250°C ausgehärtet. Nach dem Abkühlen schneidet man Prüfstäbe aus den klaren rotbraunen Platten. Die Ergebnisse der Prüfung sind in der folgenden Tabelle zusammengefasst.

Tabelle

| Anwendungsbeispiel<br>Harz nach Beispiel | I<br>1 | II<br>5 | III<br>6 | IV<br>7 |
|---|---|---|---|---|
| Biegefestigkeit nach ISO 178 ($N/mm^2$) | 110,3 | 49,9 | 48,2 | 17,5 |
| Randfaserdehnung nach ISO 178 (%) | 3,7 | 1,4 | 1,5 | 0,5 |
| Schlagbiegefestigkeit nach VSM 77105 ($kJ/m^2$) | 6,8 | 3,0 | 2,4 | 0,5 |
| Formbeständigkeit in der Wärme nach ISO 75 (°C) | >250 | >250 | >250 | 230 |
| Glasumwandlungstemperatur Tg (°C) (gemessen mit TA 2000[1]) | >300 | >300 | >300 | - |
| Wasseraufnahme nach 1 Stunde bei 100°C (%) | 0,63 | 0,41 | 0,56 | 0,51 |
| 10 % Gewichtsverlust bei °C [2]) | 460 | 437 | 450 | 450 |

[1]) TA 2000 = Differentialtheromoanalysesystem TA 2000 der Firma Mettler AG, Greifensee, CH.

[2]) Messung durch Aufheizen einer Probe in TA 2000;
Ermittlung der Temperatur, bei der 10 % der Probe verflüchtigt sind;
Aufheizgeschwindigkeit: 4°C/min (in Luft).

Anwendungsbeispiel V: Herstellung eines Laminats

Das gemäss Beispiel 1 erhaltene Bisimid wird als 40%ige Lösung in Methylenchlorid zur Imprägnierung eines C-Fasergewebes [G814 NT ex CIBA-GEIGY BSD (GB)] verwendet. Die Prepregs werden während 10 Min bie 100°C getrocknet und weisen einen Harzgehalt von 45,3 % auf.

| | | |
|---|---|---|
| - Verweilzeit in der Presse: | 3 h | Vorhärtung |
| - Temperatur: | 160°C | |
| - Druck: | 0,5 kp/mm² | |

| | | |
|---|---|---|
| - Härtungstemperatur: | 220°C | Härtung |
| - Härtungszeit: | 6 h | |
| - Druck während der Härtung: | 25-30 kp/mm² | |

Die Laminate weisen ein Faservolumen nach IS 341/BSD von $V_f$ = 58,5 % auf.

An den Laminatprobekörpern (55 x 5 x 2,5 mm) wird eine sehr hohe Glasumwandlungstemperatur von >350°C und eine ausgezeichnete thermische Beständigkeit festgestellt:

Interlaminare Scherfestigkeit nach

| | |
|---|---|
| ASTM D 2344 (N/mm²): | 30,4 bei 23°C |
| | 31,8 bei 180°C |
| | 35,6 bei 230°C |

In den folgenden Beispielen 8 bis 10 wird die Herstellung eines reinen Bisimids der Formel I beschrieben.

Beispiel 8:

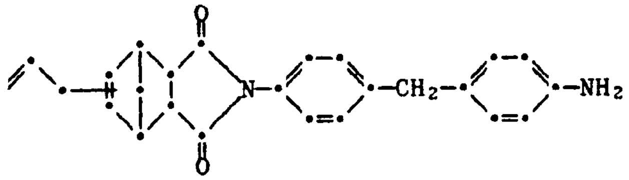

204 g Allyl-bicyclo[2.2.1]hept-5-en-2,3-dicarbonsäureanhydrid (hergestellst gemäss US 3,105,839) und 297 g 4,4'-Diaminodiphenylmethan werden in 750 ml Toluol vorgelegt und 4 Stunden am Wasserabscheider unter Rückfluss erhitzt. Die Reaktionslösung wird auf Raumtemperatur abgekühlt und unter Rühren mit 2 l 2N Salzsäure versetzt. Dabei entsteht eine dreiphasige Mischung. Die unterste Phase, die weder mit Toluol noch mit Wasser mischbar ist, wird abgetrennt und zweimal mit je 200 ml Toluol gewaschen. Anschliessend wird mit 1N NaOH alkalisiert und dann die freie Base mit Toluol (2 x 500 ml) extrahiert. Die Toluolphasen werden über Natriumsulfat getrocknet, filtriert und das Lösungsmittel bei 80°C am Rotationsverdampfer abdestilliert. Man erhält 255 g eines roten Festharzes mit einem Schmelzpunkt von 61-65°C und einem $NH_2$-Gehalt von 2,48 Mol/kg (95,5 % der Theorie).

Analyse:

| | % C | % H | % N |
|---|---|---|---|
| berechnet für $C_{25}H_{24}N_2O_2$ | 78,10 | 6,29 | 7,29 |
| gefunden | 77,85 | 6,26 | 7,10 |

Beispiel 9:

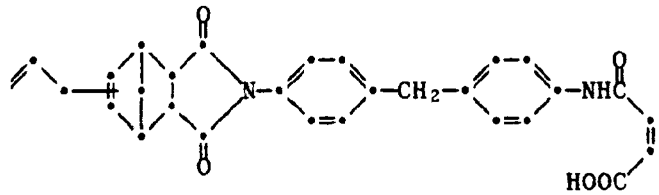

Eine Lösung von 40 g Maleinsäureanhydrid und 153,6 g Amin (hergestellt gemäss Beispiel 8) in 600 ml Aceton wird 16 Stunden bei Raumtemperatur gerührt. Der Niederschlag wird abfiltriert, mit 50 ml Aceton gewaschen und im Vakuumschrank bei 50°C getrocknet. Man erhält 134 g (70 % der Theorie) Amidsäure als weisses Pulver mit einem Schmelzpunkt >250°C und einem -COOH-Gehalt von 2,10 Mol/kg (100 % der Theorie).

Analyse:

| | % C | % H | % N |
|---|---|---|---|
| berechnet für $C_{29}H_{26}N_2O_5$ | 72,19 | 5,43 | 5,81 |
| gefunden | 71,86 | 5,40 | 5,80 |

Beispiel 10:

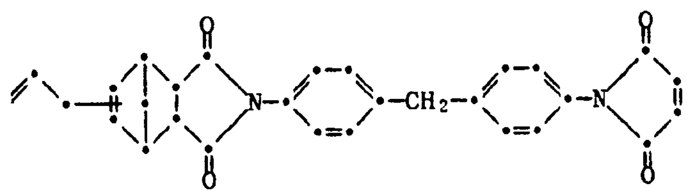

Zu einer Lösung von 4,8 g Natriumacetat in 50 ml Essigsäureanhydrid gibt man bei 70 -75°C innert 30 Minuten portionenweise 48,2 g Amidsäure (hergestellt gemäss Beispiel 9) zu. Anschliessend wird 60 Minuten bei 80°-85°C nachgerührt. Dann destilliert man den Ueberschuss an Essigsäureanhydrid ab und nimmt den Rückstand in 200 ml Methylenchlorid auf. Man wäscht zweimal mit je 100 ml 1N NaOH, mit Wasser und trocknet über Natriumsulfat. Nach Abdampfen des Methylenchlorids erhält man 41,5 g (90 % der Theorie) eines gelben Festharzes mit einem Erweichungspunkt von 71°C und einem durch Gelpermeationschromatographie (THF) ermittelten Molekulargewicht von 445 ($\overline{M}_n$) bzw. 452 ($\overline{M}_w$).

| Analyse: | % C | % H | % N |
|---|---|---|---|
| berechnet für $C_{29}H_{24}N_2O_4$ | 74,98 | 5,21 | 6,03 |
| gefunden | 73,74 | 5,28 | 5,93 |

Anwendungsbeispiel VI

Das gemäss Beispiel 10 hergestellte Festharz giesst man als heisses, dünnflüssiges Harz in ein Reagenzglas und härtet 2 Stunden bei 140°C, 2 Stunden bei 180°C, 1 Stunde bei 200°C und 6 Stunden bei 250°C. Man erhält einen transparenten, rotbraunen Festkörper mit einer Glasumwandlungstemperatur (gemessen mittels TMA)[1]1 von 378°C.

Beispiel 11: Diels-Alder Addukte aus Allylcyclopentadien und N,N',4,4'-Diphenylmethan-bismaleinimid

a) Verwendet man anstelle der in Beispiel 1 eingesetzten Mengen auf 1 mol N,N',4,4'-Diphenylmethanbismaleinimid 0,4 mol Allylcyclopentadien und geht im übrigen in gleicher Weise wie in Beispiel 1 beschrieben vor, so erhält man in quantitativer Ausbeute ein rotbraunes Festharz mit einem Erweichungspunkt von 45°C. Bei der Differentialthermoanalyse (System TA 2000 der Fa. Mettler AG, Greifensee, CH) zeigt das Harz einen Reaktionsbeginn $T_A$ bei 130°C und ein Reaktionsmaximum $T_{max}$ bei 211°C. Die integrale Reaktionswärme $\Delta H$ beträgt 240 kJ/kg.

b) Verwendet man anstelle der in Beispiel 1 eingesetzten Mengen auf 1 mol N,N',4,4'-Diphenylmethanbismaleinimid 1,6 mol Allylcyclopentadien und geht im übrigen in gleicher Weise wie in Beispiel 1 beschrieben vor, so erhält man in quantitativer Ausbeute ein rotbraunes Festharz mit einem Erweichungspunkt von 50°C. Bei der Differentialthermoanalyse zeigt das Harz ein Reaktionsbeginn $T_A$ bei 220°C und ein Reaktionsmaximum $T_{max}$ bei 297°C. Die integrale Reaktionswärme begträgt 239 kJ/kg.

Anwendungsbeispiele VII und VIII

Die gemäss Beispiel 11a und 11b hergestellten Festharze giesst man als heisses, dünnflüssiges Harz in ein Reagenzglas und härtet 3 Stunden bei 160°C, 1 Stunde bei 200°C und 6 Stunden bei 250°C. Man erhält transparente, rotbraune Festkörper mit Glasumwandlungstemperaturen > 300°C.

**Patentansprüche**

1. Bisimide der Formel I

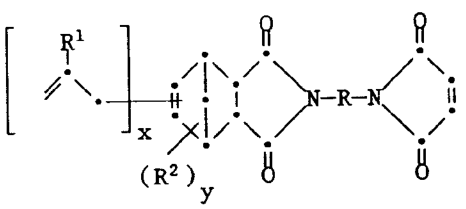

(I) ,

worin x Null, 1, 2 oder 3, y Null oder eine ganze Zahl von 1 bis 6 und $x + y \leqq 6$ sind, $R^1$ Wasserstoff oder Methyl und $R^2$ Chlor, Brom, $C_1$-$C_4$-Alkyl, Phenyl oder Benzyl bedeuten und R für $-C_mH_{2m}-$ mit m = 2-20, Cycloalkylen mit 5 bis 10 C-Atomen, Bis(methylen)cycloalkylen mit 7 bis 12 C-Atomen, Arylen mit 6 bis 10 C-Atomen, Bis(methylen)arylen mit 8 bis 12 C-Atomen oder für eine Gruppe der Formel II

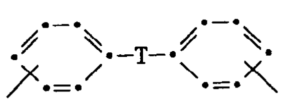

(II) ,

worin T Methylen, Isopropyliden, CO, O, S oder $SO_2$ bedeutet, steht, wobei Bisimide der Formel I, in welcher x Null, y 6, $R^2$ Brom und R para-Phenylen oder eine Gruppe der Formel II sind, worin T O, S, $SO_2$ oder $-CH_2-$ bedeutet, ausgenommen sind.

2. Bismide der Formel I nach Anspruch 1, worin $R^1$ Wasserstoff, x 1 und y Null bedeuten.

1(Du Pont 9900 Thermal Analyser)

3. Bismide der Formel I nach Anspruch 1, worin $R^2$ $C_1$-$C_3$-Alkyl oder Benzyl, y 1 und x Null bedeuten.

4. Bismide der Formel I nach Anspruch 1, worin R $-(CH_2)_p-$ mit p = 2=12, cyclohexylen, Bis(methylen)cyclohexan, Bis(cyclohexylen)methan, Phenylen, Xylylen oder eine Gruppe der Formel II nach Anspruch 1, worin T für Methylen, O oder $SO_2$ steht, bedeudet.

5. Bismide der Formel I nach Anspruch 4, worin R 1,4-Phenylen, 1,4-Xylylen oder Bis(1,4-phenylen)methan bedeutet.

6. Stoffgemische enthaltenf einem Bismid der Formel I nach Anspruch 1, erhältlich durch Umsetzung eines Bismaleinimids der Formel III

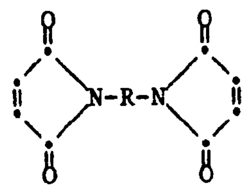

(III)

mit 0,4 bis 1,6 Aequivalenten eines Cyclopentadiens der Formel IV

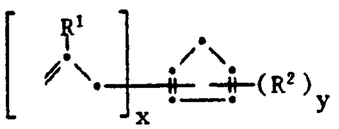

(IV),

wobei die Symbole R, $R^1$, $R^2$, x und y die im Anspruch 1 angegebene Bedeutung haben.

7. Stoffgemische nach Anspruche 6, worin pro Bismaleinimid der Formel III 0,7 bis 1,3 Aequivalente des Cyclopentadiens der Formel IV eingesetzt werden.

8. Stoffgemische nach Anspruch 6, enthaltend zusätzlich 1 bis 30 Gew.-%, bezogen auf das Gesamtgemisch, einer zur Reaktion mit dem Bisimid der Formel I nach Anspruch 1 oder mit dem Bismaleinimid der Formel III nach Anspruch 6 befähigten Verbindung.

9. Stoffgemische nach Anspruch 8, die als zusätzliche Verbindung ein Diamin oder Diol der Formel IX

$$HQ\text{-}R'\text{-}QH \quad (IX),$$

worin Q O oder NH und R′ einen zweiwertigen organischen Rest mit 2-30 C-Atomen bedeuten, oder einen Phenol- oder Kresol-Novolak oder Gemische solcher Verbindungen enthalten.

10. Stoffgemische nach Anspruch 8, die als zusätzliche Verbindung eine Verbindung der Formel X

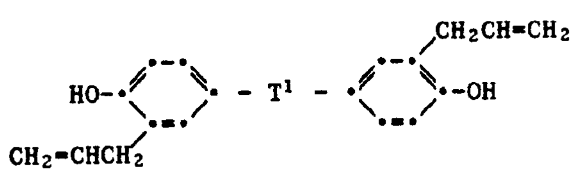

(X)

enthalten, worin $T^1$ die direkte Bindung, Methylen, Isopropyliden, O, S, SO oder $SO_2$ bedeutet.

11. Stoffgemische nach Ansprüchen 9 und 10, die als zusätzliche Verbindung einen Phenol- oder Kresol-Novolak, eine Verbindung der Formel IXa

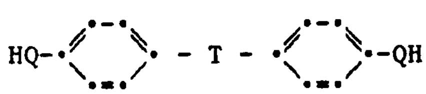

(IXa)

oder eine Verbindung der Formel X oder ein Gemisch der genannten Verbindungen enthalten, worin Q O oder NH, T Methylen oder Isopropyliden und $T^1$ Isopropyliden bedeuten.

12. Polymere, die dadurch erhältlich sind, dass man ein Bisimid der Formel I nach Anspruch 1 während 1 bis 20 Stunden auf eine Temperatur zwischen 100 und 300°C erhitzt.

## Claims

1. A bisimide of the formula I

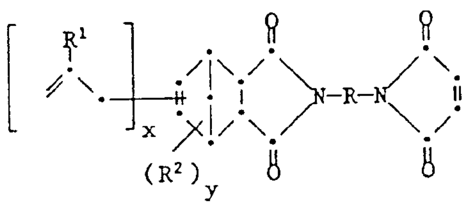

(I),

in which x is zero, 1, 2 or 3, y is zero or an integer from 1 to 6 and $x + y \leqq 6$, $R^1$ is hydrogen or methyl and $R^2$ is chlorine, bromine, $C_1$-$C_4$alkyl, phenyl or benzyl and R is $-C_mH_{2m}-$ in which m = 2-20, cycloalkylene having 5 to 10 C atoms, bis(methylene)cycloalkylene having 7 to 12 C atoms, arylene having 6 to 10 C atoms, bis-(methylene)arylene having 8 to 12 C atoms or a group of the formula II

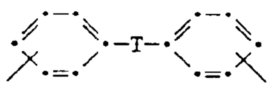

(II),

in which T is methylene, isopropylidene, CO, O, S or $SO_2$, with the exception of bisimides of the formula I in which x is zero, y is 6, $R^2$ is bromine and R is para-phenylene or a group of the formula II in which T is O, S, $SO_2$ or $-CH_2-$.

2. A bisimide of the formula I, according to claim 1, in which $R^1$ is hydrogen, x is 1 and y is zero.

3. A bisimide of the formula I, according to claim 1, in which $R^2$ is $C_1$-$C_3$alkyl or benzyl, y is 1 and x is zero.

4. A bisimide of the formula I, according to claim 1, in which R is $-(CH_2)_p-$ in which p = 2-12, cyclohexylene, bis(methylene)cyclohexane, bis(cyclohexylene)methane, phenylene, xylylene or a group of the formula II, according to claim 1, in which T is methylene, O or $SO_2$.

5. A bisimide of the formula I, according to claim 4, in which R is 1,4-phenylene, 1,4-xylylene or bis(1,4-phenylene)methane.

6. A composition of matter containing a bisimide of the formula I, according to claim 1, obtainable by reacting a bismaleimide of the formula III

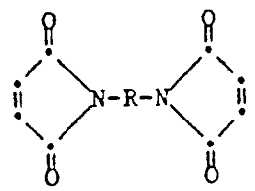

(III)

with 0.4 to 1.6 equivalents of a cyclopentadiene of the formula IV

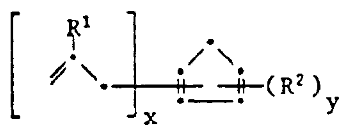

(IV),

in which the symbols R, $R^1$, $R^2$, x and y are as defined in claim 1.

7. A composition of matter according to claim 6, in which 0.7 to 1.3 equivalents of the cyclopentadiene of the formula IV are employed per bismaleimide of the formula III.

8. A composition of matter according to claim 6, containing, in addition, 1 to 30% by weight, relative to the total mixture, of a compound capable of reacting with the bisimide of the formula I, according to claim 1, or with the bismaleimide of the formula III, according to claim 6.

9. A composition of matter according to claim 8, containing, as the additional compound, a diamine or diol

of the formula IX

HQ-R'-QH (IX)

in which Q is O or NH and R' is a divalent organic radical having 2-30 C atoms, or a phenol or cresol novolak or mixtures of such compounds.

10. A composition of matter according to claim 8, containing, as the additional compound, a compound of the formula X

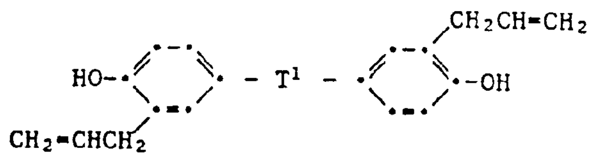

(X)

in which $T^1$ is a direct bond, methylene, isopropylidene, O, S, SO or $SO_2$.

11. A composition of matter according to claims 9 and 10, containing, as the additional compound, a phenol or cresol novolak, a compound of the formula IXa

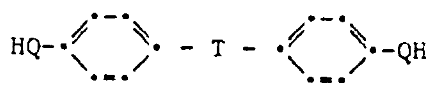

(IXa)

or a compound of the formula X or a mixture of the said compounds, Q being O or NH, T being methylene or isopropylidene and $T^1$ being isopropylidene.

12. A polymer which can be obtained by heating a bisimide of the formula I, according to claim 1, at a temperature between 100 and 300°C for 1 to 20 hours.

## Revendications

1. Bis-imides répondant à la formule I :

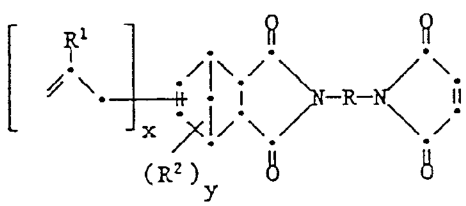

(I)

dans laquelle x est égal à 0, à 1, à 2 ou à 3, y désigne un nombre entier de 0 à 6, la somme (x+y) étant au plus égale à 6, $R^1$ représente l'hydrogène ou un-méthyle, $R^2$ représente le chlore, le brome, un alkyle en $C_1$-$C_4$ ou un benzyle et R représente un radical $-C_mH_{2m}-$ dans lequel m désigne un nombre de 2 à 20, un radical cycloalkylène contenant de 5 à 10 atomes de carbone, un radical bis-(méthylène) cycloalkylène contenant de 7 à 12 atomes de carbone, un radical arylène contenant de 6 à 10 atomes de carbone, un radical bis-(méthylène)-arylène contenant de 8 à 12 atomes de carbone ou un radical répondant à la formule II :

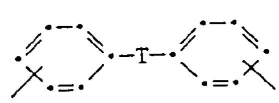

(II)

dans laquelle T représente un méthylène, un isopropylidène, CO, O, S ou $SO_2$, sauf les bis-imides de formule I dans lesquels x est égal à 0, y est égal à 6, $R^2$ représente le brome et R représente un radical p-phénylène ou un radical de formule II dans lequel T représente O, S, $SO_2$ ou $-CH_2-$.

2. Bis-imides de formule I selon la revendication 1 dans lesquels $R^1$ représente l'hydrogène, x est égal à 1 et y à 0.

3. Bis-imides de formule I selon la revendication 1 dans lesquels $R^2$ représente un alkyle en $C_1$-$C_3$ ou un benzyle, y est égal à 1 et x à 0.

4. Bis-imides de formule I selon la revendication 1 dans lesquels R représente un radical $-(CH_2)_p-$ dont l'indice p est un nombre de 2 à 12, un radical cyclohexylène, bis-(méthylène)-cyclohexane, bis-(cyclohexylène)-méthane, phénylène ou xylylène ou un radical de formule II selon la revendication 1 dans lequel T représente un radical méthylène, O ou $SO_2$.

5. Bis-imides de formule I selon la revendication 4 dans lesquels R représente un radical 1,4-phénylène, 1,4-xylylène ou bis-(1,4-phénylène)-méthane.

6. Mélanges contenant un bis-imide de formule I selon la revendication 1 qui peuvent être obtenus par réaction d'un bis-maléimide répondant à la formule III :

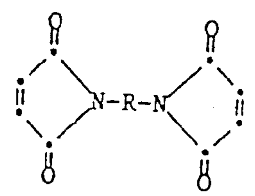

avec de 0,4 à 1,6 équivalent d'un cyclopentadiène répondant à la formule IV :

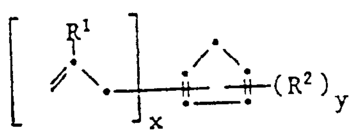

(IV),

formules dans lesquelles R, $R_1$, $R_2$, x et y ont les significations qui leur ont été données à la revendication 1.

7. Mélanges selon la revendication 6 dans lesquels on utilise, par bis-maléimide de formule III, de 0,7 à 1,3 équivalent du cyclopentadiène de formule IV.

8. Mélanges selon la revendication 6 qui contiennent en outre de 1 à 30% en poids, par rapport au mélange total, d'un composé capable de réagir avec le bisimide de formule I selon la revendication 1 ou avec le bis-maléimide de formule III selon la revendication 6.

9. Mélanges selon la revendications 8 qui contiennent, comme composé supplémentaire, une diamine ou un diol répondant à la formule IX :

$$HQ\text{-}R'\text{-}QH \quad (IX)$$

dans laquelle Q représente O ou NH et R' représente un radical organique divalent contenant de 2 à 30 atomes de carbone, ou une novolaque phénolique ou crésolique ou un mélange de tels composés.

10. Mélanges selon la revendication 8 qui contiennent, comme composé supplémentaire, un composé répondant à la formule X :

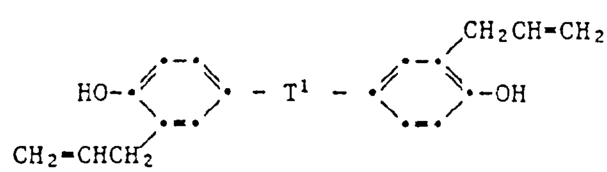

dans laquelle $T^1$ représente une liaison directe, un méthylène, un isopropylidène, O, S, SO ou $SO_2$.

11. Mélanges selon l'une des revendications 9 et 10 qui contiennent, comme composé supplémentaire, une novolaque phénolique ou crésolique, un composé de formule IXa :

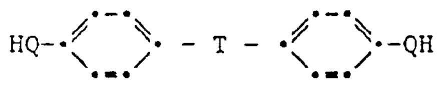

ou un composé de formule X, ou un mélange des composés cités, dans lesquels Q représente O ou NH, T représente un radical méthylène ou isopropylidène et $T^1$ représente un radical isopropylidène.

12. Polymères qui peuvent être obtenus par chauffage d'un bis-imide de formule I selon la revendication 1 à une température de 100 à 300°C pendant une durée de 1 à 20 heures.